# EUROPEAN PATENT APPLICATION

(11) **EP 4 667 020 A1**
(43) Date of publication of application: **24.12.2025**
(21) Application number: 24755899.2
(22) Date of filing: 19.01.2024
(51) Int. Cl.: A61K 51/06, A61P 35/00, A61K 103/00, A61K 103/32, A61K 103/20, A61K 101/02, A61K 103/30, A61K 103/10, A61K 103/34, A61K 103/36

(54) **RADIONUCLIDE-LABELED BORON-CONTAINING DRUG, AND PREPARATION AND USE THEREOF**

(30) Priority: 17.02.2023 CN 202310131162
(71) Applicant: Chengdu New Radiomedicine Technology Co., Ltd., Chengdu, Sichuan 610200 (CN)
(72) Inventor: QIAN, Zhiyong, Chengdu, Sichuan 610000 (CN); DAI, Liqun, Chengdu, Sichuan 610213 (CN); ZHAO, Xiaosheng, Ganzhou, Jiangxi 341000 (CN); BAI, Bing, Chengdu, Sichuan 610200 (CN); MA, Wenliang, Chengdu, Sichuan 610213 (CN); LU, Jing, Chongqing 408500 (CN); GE, Qiang, Chengdu, Sichuan 610000 (CN); CAI, Jiming, Chengdu, Sichuan 610000 (CN); LIU, Fang, Chengdu, Sichuan 610041 (CN); TAN, Wenjing, Chengdu, Sichuan 610213 (CN)
(74) Representative: Barker Brettell LLP
(86) International application number: PCT/CN2024/073240
(87) International publication number: WO 2024/169524

(57) **Abstract**

The present invention relates to the field of radiopharmaceuticals, and in particular to a radionuclide-labeled boron-containing drug, and a preparation method therefor. The radionuclide-labeled boron-containing drug is obtained by labeling a boron-containing drug by at least one radionuclide; the boron-containing drug is obtained by polymerizing dopamine and a derivative thereof with a boron-containing compound; the radionuclide-labeled boron-containing drug has tumor targeting characteristics. The present invention provides a method for implementing labeling of a boron-containing drug by radionuclides by means of the technical route of complexation or covalent bonds.

## Description

### TECHNICAL FIELD

The present invention relates to the field of radiopharmaceuticals, and in particular to a radionuclide-labeled boron-containing drug, and a preparation method and use therefor.

### BACKGROUND

Local targeted internal radiation therapy is an internal radiation therapy that delivers radionuclide accurately into the local area of the tumor through a carrier so as to achieve treatment by the radiation emitted by the radionuclide. Due to its outstanding therapeutic effects, it plays an important role in the treatment of diseases such as cancer and has become one of the hot research directions. Currently, there are methods, technologies, or drugs that use microspheres, sealed metal seed sources, small molecules, antibodies, polypeptides, and nanomedicines as carriers for radionuclides to achieve precise local internal radiation therapy. The most challenging technical difficulties in the development of targeted radiopharmaceuticals are: 1. Designing and developing carriers with good targeting properties; 2. Achieving efficient and stable labeling of radionuclides on carriers with good targeting properties, and ensuring that the labeled carriers maintain the ability to target lesions or tumor cells effectively.

At present, most of the targeted drug carriers such as small molecules, antibodies, and polypeptides in clinical applications face difficulties in achieving efficient and stable radionuclide labeling, poor stability after labeling, and loss of targeting properties after labeling. It is precisely because of the high technical difficulty that the development of targeted radiopharmaceuticals has progressed relatively slowly and lagged behind.

### SUMMARY

To address the technical problem in the background, an object of the present invention is to provide a radionuclide-labeled boron-containing drug, and a preparation method and use therefor. The radiopharmaceutical can be labeled with radionuclides such as ⁶⁸Ga, ⁶⁷Ga, ⁶⁴Cu, ⁶⁷Cu, ⁸⁹Zr, ⁸⁹Sr, ⁹⁰Y, ¹⁷⁷Lu, ¹¹¹In, ¹⁶⁵Dy, ¹⁶⁶Ho, ²⁰¹TI, ²¹³Bi, ²¹²Bi, ²²⁵Ac, ²²³Ra, ²¹²Pb, ²¹¹At, ¹²⁴I, ¹²³I, ¹²⁵I, ¹³¹I, ¹⁸F, ¹⁸⁶Re, ¹⁸⁸Re, and ^{99m}Tc, achieving integration of diagnosis and treatment.

The first technical solution provided by the invention is:
A radionuclide-labeled boron-containing drug which is obtained by labeling a boron-containing drug by at least one radionuclide;
the boron-containing drug is obtained by polymerizing dopamine and its derivative with a boron-containing compound.

Preferably, the radionuclide comprises any one or more of ⁶⁸Ga, ⁶⁷Ga, ⁶⁴Cu, ⁶⁷Cu, ⁸⁹Zr, ⁸⁹Sr, ⁹⁰Y, ¹⁷⁷Lu, ¹¹¹In, ¹⁶⁵Dy, ¹⁶⁶Ho, ²⁰¹TI, ²¹³Bi, ²¹²Bi, ²²⁵Ac, ²²³Ra, ²¹²Pb, ²¹¹At, ¹²⁴I, ¹²³I, ¹²⁵I, ¹³¹I, ¹⁸F, ¹⁸⁶Re, ¹⁸⁸Re, and ^{99m}Tc.

Preferably, the radionuclide-labeled boron-containing drug has a particle size of 50-1000 nm.

Preferably, the boron-containing compound includes, but is not limited to, any one or more of boric acid, phenylalanine boric acid and its derivative, proline boric acid, aspartic acid boric acid, tyrosine boric acid, cysteine boric acid, methionine boric acid, serine boric acid, 5-amino-2,3-difluorophenyl boric acid, 3-amino-5-cyanophenyl boric acid, 3-amino-4-methylphenyl boric acid, 3-amino-phenylboric acid hydrochloride, and 4-carbamoyl phenylboric acid.

Preferably, the dopamine and its derivative comprise any one or more of dopamine, 4-(2-aminopropyl)phenyl-1,2-diol, norepinephrine (1-(3,4-hydroxyphenyl)-2-amino ethanol), L-methyldopa (2-methyl-3-(3,4-dihydroxyphenyl)-L-alanine), droxidopa ((2S,3R)-(2-amino-3-(3,4-dihydroxyphenyl)-3-hydroxy acrylic acid), and 5-hydroxydopamine.

Preferably, the dopamine and its derivative are dopamine, L-methyldopa, and 5-hydroxydopamine.

Preferably, the weight ratio of the dopamine and its derivative to the boron-containing compound is (1-10) : 1.

The radionuclide-labeled boron-containing drug according to any one of claims 1-7, wherein an indication for the radionuclide-labeled boron-containing drug comprises head and neck tumors, lung cancer, peritoneal cancer, liver cancer, gastric cancer, melanoma, pancreatic cancer, glioblastoma, cervical cancer, ovarian cancer, bladder cancer, breast cancer, colon cancer, colorectal cancer, uterine cancer, salivary gland cancer, kidney cancer, prostate cancer, vulvar cancer, or thyroid cancer.

The second technical solution provided by the invention is:

A method for preparing a radionuclide-labeled boron-containing drug, comprising steps of:
performing complexation of polyhydroxy or carboxyl groups on a boron-containing drug with a radionuclide, and performing precipitation and solidification; or
labeling a radionuclide in a phenyl ring structure of boron-containing drug by a formation of covalent bonds through chemical reactions.

Compared with the prior art, the present invention has the following beneficial effects:
The radiopharmaceutical of the present invention achieves efficient labeling of radionuclides such as ⁶⁸Ga, ⁶⁷Ga, ⁶⁴Cu, ⁶⁷Cu, ⁸⁹Zr, ⁸⁹Sr, ⁹⁰Y, ¹⁷⁷Lu, ¹¹¹In, ¹⁶⁵Dy, ¹⁶⁶Ho, ²⁰¹TI, ²¹³Bi, ²¹²Bi, ²²⁵Ac, ²²³Ra, ²¹²Pb, ²¹¹At, ¹²⁴I, ¹²³I, ¹²⁵I, ¹³¹I, ¹⁸F, ¹⁸⁶Re, ¹⁸⁸Re, and ^{99m}Tc. The labeling rates of multiple radionuclides such as ⁶⁸Ga, ⁶⁷Ga, ⁹⁰Y, ¹⁷⁷Lu, ¹⁶⁵Dy, and ¹⁶⁶Ho exceed 98%. The radiopharmaceutical exhibits good stability in normal saline, phosphate buffer, and 5% fetal bovine serum. The radiopharmaceutical of the invention has a particle size of 50-1000 nm, good stability, can enter cells, penetrate the blood-brain barrier, and possess tumor targeting properties. It can be used simultaneously for tumor treatment or imaging, and the imaging radionuclide-labeled drug can be used for the study of drug distribution and the screening or prediction of treatment effects in potential patients.

### DESCRIPTION OF THE DRAWINGS

Fig. 1 is a SEM image of the boron-containing drug after reconstitution in Example 2;
Fig. 2 is a SEM image of the ⁹⁰Y-labeled boron-containing drug in Example 4;
Fig. 3 is a DLS image of the ⁹⁰Y-labeled boron-containing drug in Example 4;
Fig. 4 is an in vivo SPECT-CT experimental image of the ¹³¹I-labeled boron-containing drug in mice in Example 12;
Fig. 5 is an in vivo SPECT-CT experimental image of free ¹³¹I in mice in Example 12.

### DETAILED DESCRIPTION

To better understand the technical solution of the present invention, the following description will be made in conjunction with the accompanying drawings and examples. The ways to implement the present invention include but are not limited to the following examples, which are provided to illustrate the invention but are not intended to limit the scope of the invention. Unless otherwise specified, the technical means used in the examples are conventional means known to those skilled in the art. The experimental methods in the following examples are conventional unless otherwise indicated.

The first embodiment of the present invention provides a radionuclide-labeled boron-containing drug which is obtained by labeling a boron-containing drug by at least one radionuclide; wherein the boron-containing drug is obtained by polymerizing dopamine and its derivative with a boron-containing compound.

The radionuclide labeling of the boron-containing drug is realized by complexation or covalent bond between the boron-containing drug and the radionuclide.

The molecular structure of the boron-containing drug of the present invention contains polyphenols, carboxyl groups, and phenyl ring structures, which exhibit good tumor targeting properties. Moreover, the boron-containing drug itself possesses good physicochemical stability, and after being labeled with radionuclides, it can become a radioactive drug with good targeting properties. The boron-containing drug itself does not have imaging properties, and the study of its distribution and metabolism in the body is a difficult point in the drug development process. The radionuclide-labeled boron-containing drug provided by the present invention can achieve efficient and stable labeling of multiple radionuclides and still maintain good physicochemical properties and tumor targeting properties after labeling, showing potential to be developed into a drug for integration of localized targeted internal radiation diagnosis and treatment. Additionally, the imaging radionuclide-labeled boron-containing drug can help study the drug's distribution and metabolism in the body, providing assistance for the development of the boron-containing drug.

The radiopharmaceuticals of the present invention do not alter the tumor targeting properties of the boron-containing drug and can be metabolized (biodegradable) in the body. After injection into the body for 48 hours, the drug concentration rapidly decreases until it is completely metabolized and cleared.

The radionuclides used for labeling in the embodiments of the present invention include any one or more of ⁶⁸Ga, ⁶⁷Ga, ⁶⁴Cu, ⁶⁷Cu, ⁸⁹Zr, ⁸⁹Sr, ⁹⁰Y, ¹⁷⁷Lu, ¹¹¹In, ¹⁶⁵Dy, ¹⁶⁶Ho, ²⁰¹TI, ²¹³Bi, ²¹²Bi, ²²⁵Ac, ²²³Ra, ²¹²Pb, ²¹¹At, ¹²⁴I, ¹²³I, ¹²⁵I, ¹³¹I, ¹⁸F, ¹⁸⁶Re, ¹⁸⁸Re, and ^{99m}Tc.

The radionuclide-labeled boron-containing drug typically has a particle size of 50-1000 nm and has the properties of being taken up by cancer cells to achieve tumor targeting.

In some preferred embodiments, the boron-containing compound includes, but is not limited to, any one or more of boric acid, phenylalanine boric acid and its derivative, proline boric acid, aspartic acid boric acid, tyrosine boric acid, cysteine boric acid, methionine boric acid, serine boric acid, 5-amino-2,3-difluorophenyl boric acid, 3-amino-5-cyanophenyl boric acid, 3-amino-4-methylphenyl boric acid, 3-amino-phenylboric acid hydrochloride, and 4-carbamoyl phenylboric acid.

In some preferred embodiments, the dopamine and its derivative comprise any one or more of dopamine, 4-(2-aminopropyl)phenyl-1,2-diol, norepinephrine (1-(3,4-hydroxyphenyl)-2-amino ethanol), L-methyldopa (2-methyl-3-(3,4-dihydroxyphenyl)-L-alanine), droxidopa ((2S,3R)-(2-amino-3-(3,4-dihydroxyphenyl)-3-hydroxy acrylic acid), and 5-hydroxydopamine; preferably, dopamine, L-methyldopa (2-methyl-3-(3,4-dihydroxyphenyl)-L-alanine) or 5-hydroxydopamine; preferably, dopamine, L-methyldopa, or 5-hydroxydopamine.

In some preferred embodiments, the weight ratio of the dopamine and its derivative to the boron-containing compound is (1-10): 1.

The indication for the radionuclide-labeled boron-containing drug of the present invention comprises diseases such as, head and neck tumors, lung cancer, peritoneal cancer, liver cancer, gastric cancer, melanoma, pancreatic cancer, glioblastoma, cervical cancer, ovarian cancer, bladder cancer, breast cancer, colon cancer, colorectal cancer, uterine cancer, salivary gland cancer, kidney cancer, prostate cancer, vulvar cancer, or thyroid cancer.

The second embodiment of the present invention provides a method for preparing the radionuclide-labeled boron-containing drug, which can be achieved in two ways: ① complexation of the metal radionuclide with the polyhydroxyl and carboxyl groups on the boron-containing drug, and further precipitation and solidification, so as to achieve stable labeling; and ②labeling a radionuclide in a phenyl ring structure of boron-containing drug by a formation of covalent bonds through chemical reactions so as to achieve stable labeling. There is no specific limitation on the labeling method, as long as the above two ways of labeling can be achieved.

As some specific embodiments, the method of labeling radionuclides on boron-containing drugs through complexation is at least applicable to radionuclides such as ⁶⁸Ga, ⁶⁷Ga, ⁶⁴Cu, ⁶⁷Cu, ⁸⁹Zr, ⁸⁹Sr, ⁹⁰Y, ¹⁷⁷Lu, ¹¹¹In, ¹⁶⁵Dy, ¹⁶⁶Ho, ²⁰¹TI, ²¹³Bi, ²¹²Bi, ²²⁵Ac, ²²³Ra, and ²¹²Pb. The specific method is as follows: weighing 10-100mg of boron-containing drug, adding it to a buffer solution with a pH of 3.0-7.0, and mixing uniformly with ultrasound; adding 0.1-500mCi of radionuclide solution to the ultrasonic treated solution, and reacting in a water bath of 30-80°C for 5-60 minutes to allow the boron-containing drug to fully combine with the radionuclide to obtain a fully combined mixture; centrifuging the fully combined mixture to obtain a solid, washing the solid three times with a buffer solution, collecting the supernatant and precipitate, and measuring their radioactive activities respectively, and calculating the labeling rate of the radionuclide-labeled boron-containing drug; placing a certain activity of radionuclide-labeled boron-containing drug in normal saline, phosphate buffer, and 5% fetal bovine serum, respectively, and incubating at 37°C for a period of time; and measuring the activity or radioactive counting of the supernatants to calculate the radionuclide leakage rates of the drug in different solutions. After testing, the labeling rates of radionuclides such as ⁶⁸Ga, ⁶⁷Ga, ⁹⁰Y, ¹⁷⁷Lu, ¹⁶⁵Dy, and ¹⁶⁶Ho are greater than 98%, and the leakage rates of radionuclides in normal saline, phosphate buffer, and 5% fetal bovine serum are less than 5% after 6 days.

The method of labeling radionuclides on boron-containing drugs through complexation is also applicable to radionuclides such as ¹⁸⁶Re, ¹⁸⁸Re, and ^{99m}Tc. The specific method is as follows: Sequentially adding 4-500mg of sodium ascorbate and 0.1-100mCi of Nₐ¹⁸⁶ReO₄/Na¹⁸⁸ReO₄/Na^{99m}TcO₄ to a sodium acetate buffer solution containing 5-100mg of nanoparticles (pH 3.0-6.0), and shaking at 80°C for 15 minutes, centrifuging to obtain a solid, wash the solid three times with a buffer solution, collecting the supernatant and precipitate, and measuring their radioactive activities respectively, calculating the labeling rate of the radionuclide-labeled boron-containing drug. After testing, the labeling rates of ¹⁸⁶Re, ¹⁸⁸Re, and ^{99m}Tc are greater than 85%, and the radioactive drugs of the present invention can be used for tumor treatment.

As other specific embodiments, the method of labeling radionuclides on boron-containing drugs through covalent reaction is at least applicable to radionuclides such as ²¹¹At, ¹²⁴I, ¹²³I, ¹²⁵I, and ¹³¹I. The specific method is as follows: dissolving chloramine T in an organic solvent, applying to the bottom of the tube, and drying; placing 5-10mg of boron-containing drug and a buffer solution with a pH of 7.0-8.0 in a reaction tube, placing the reaction tube in an ice bath, and adding radionuclide to react for 5-30 minutes to obtain a reaction mixture; centrifuging the reaction mixture to separate a precipitate from a supernatant, washing the precipitate multiple times, and measuring the total radioactive activity in the supernatant and precipitate respectively, and calculating the labeling rate of the radionuclide-labeled boron-containing drug; placing a certain activity of the drug in normal saline, phosphate buffer, and 5% fetal bovine serum, respectively, and incubating at 37°C for a period of time; and measuring the activity or radioactive counting of the supernatants to calculate the radionuclide leakage rates of the drug in different solutions. After testing, the labeling rates of ¹³¹I and ¹²³I are greater than 85%, and the labeling amount are greater than 10mCi/mg, with stable labelling, no significant shedding of radionuclides in the body, and outstanding therapeutic effects on tumors. The leakage rates of radionuclides are less than 1% after incubating in normal saline, phosphate buffer, and 5% fetal bovine serum for 48 hours.

The method of labeling radionuclides on boron-containing drugs through covalent reaction is also applicable to radionuclides such as ¹⁸F. The specific method is as follows: dispersing the boron-containing drug uniformly in trifluoroacetic acid solution, and bubbly introducing ¹⁸F₂ gas into the solution for reaction; collecting the precipitate after half an hour of reaction, measuring the radioactive activity, and calculating the labeling rate of the boron-containing drug. After testing, the labeling rate of ¹⁸F is greater than 70%, which can be used for the study of the in vivo distribution of boron-containing drugs.

For the preparation method of the boron-containing drug, the method comprises: mixing and reacting an aqueous solution of dopamine and its derivative with an aqueous solution of a boron-containing compound, separating a solid from a liquid to obtain the solid, and washing and freeze-drying the solid.

As some preferred embodiments, the mixing and reacting is performed at pH from 6 to 14, a time of the mixing and reacting is 0.5 hour to 48 hours, and a stirring speed during the reacting is over 200 RPM.

The third embodiment of the invention provides a use of a radionuclide-labeled boron-containing drug in the preparation of a medicament for tumor treatment and/or tumor imaging. That is, the drug can not only achieve tumor treatment or tumor imaging separately but can also realize an integrated drug for tumor diagnosis and treatment. The imaging radionuclide-labeled drug of the present invention can be used for the study of drug distribution and the screening or prediction of treatment effects in potential patients.

Based on the composition and structural design of the boron-containing drug, the boron-containing drug becomes a good radionuclide carrier, allowing radionuclides to be labeled through a simple method. After labeling with imaging and/or therapeutic radionuclides, the boron-containing drug can be used for the diagnosis and treatment of tumor patients.

In the invention, the radionuclide-labeled boron-containing drugs such as ²¹¹At, ¹²⁴I, ¹²³I, ¹²⁵I, ¹³¹I, and ¹⁸F, can be imaged under SPECT-CT, they are used for the study of the in vivo distribution of boron-containing drugs, and help understand the dynamic distribution information of boron-containing drugs in patients. Radionuclide-labeled boron-containing drugs such as ²¹¹At, ¹²⁴I, ¹²³I, ¹²⁵I, ¹³¹I, and ¹⁸F can be used for pre-treatment screening and efficacy prediction of patients, with the potential to achieve precision diagnosis and treatment. Moreover, the ¹³¹I-labeled drug is expected to be used for integrated tumor diagnosis and treatment applications.

After intravenous injection of the ¹³¹I-labeled boron-containing drug into the body, SPECT-CT imaging shows that the drug concentration is relatively high in the tumor at about 24 hours, indicating that the radionuclide labeling has not changed the tumor targeting of the boron-containing drug. No significant ¹³¹I enrichment was observed in the animal's thyroid, proving that the ¹³¹I-labeled drug is not enriched in the thyroid, and the ¹³¹I labeled on the drug does not shed significantly.

To make the technical solution of the invention clearer, the following examples are provided to illustrate the radionuclide-labeled boron-containing drug, the preparation method and use therefor.

### Example 1: Preparation of Boron-Containing Drug

80mg of dopamine hydrochloride was weighed into a reaction flask, 34 mL of 30% ethanol solution was added, and stirred to dissolve to obtain a solution (1); 20mg of borylphenylalanine (BPA) was weighed into a centrifuge tube, 3mL of water was added, and then 0.2mL of 2.5mol/L NaOH solution was added until BPA is completely dissolved to obtain a solution (2); the solution (1) and the solution (2) were mixed, the pH was adjusted to about 10 with NaOH, and stirring was performed at 300 RPM for 24 hours to obtain a solution (3); the solution (3) was centrifuged at 10000 RPM and washed with pure water three times to obtain a product; and the product was suspended in 6 mL of water and freeze-drying to obtain the boron-containing drug.

### Example 2: Redispersibility Experiment of Boron-Containing Drug after Freeze-Drying

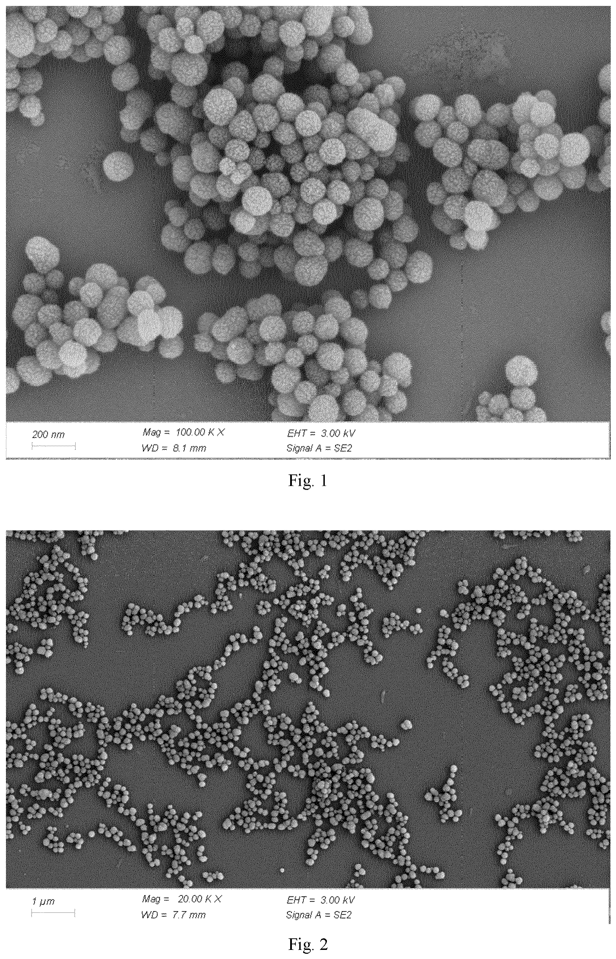

5mg of the boron-containing drug of Example 1 was taken and 5mL of solvent which includes but is not limited to one of sterile water for injection, sodium chloride injection, glucose injection, PBS buffer, and water was added as a dispersing agent. After manual mixing, SEM detection was performed. The morphology is shown in Fig. 1.

### Example 3: Preparation of Radiopharmaceuticals

This example is used to illustrate the labeling process of ⁶⁸Ga: 5mg of the boron-containing drug of Example 1 was weighed, added to 2ml of sodium acetate buffer solution at pH=5.5, and mixed uniformly with ultrasound. 500µCi of ⁶⁸GaCl₃ solution was added, and the shaking reaction was performed at 50°C in water bath for 50 minutes. Centrifugation was performed to separate a supernatant from a precipitate, the precipitate was wash 3 times with sodium acetate buffer at pH=5.5 to collect a first supernatant and a first precipitate. The radioactive activity of the first supernatant and the first precipitate were measured respectively, and the labeling rate of the boron-containing drug was calculated to be greater than 99%. The first precipitate was divided into three parts and placed in normal saline, phosphate buffer, and 5% fetal bovine serum, respectively, and incubated at 37°C for 24 hours, and then centrifuged to separate second supernatants from second precipitates. The second precipitates were washed 3 times with sodium acetate buffer at pH=5.5, to collect third supernatants and third precipitates. The radioactive activities of the third supernatants and the third precipitates were measured respectively, and the leakage rates of the boron-containing drug were calculated to be less than 1%.

### Example 4: Preparation of Radiopharmaceuticals

This example is used to illustrate the labeling process of ⁹⁰Y: 5mg of the boron-containing drug of Example 1 was weighed, added to 2ml of sodium acetate buffer solution at pH=5.5, and mixed uniformly with ultrasound. 500µCi of ⁹⁰YCI₃ standard solution was added, and the shaking reaction was performed at 50°C in water bath for 50 minutes. Centrifugation was performed to separate a supernatant from a precipitate, the precipitate was wash 3 times with sodium acetate buffer at pH=5.5 to collect a first supernatant and a first precipitate. The radioactive activity of the first supernatant and the first precipitate were measured respectively, and the labeling rate of the boron-containing drug was calculated to be greater than 98%. The first precipitate was divided into three parts and placed in normal saline, phosphate buffer, and 5% fetal bovine serum, respectively, and incubated at 37°C for 6 days, and then centrifuged to separate second supernatants from second precipitates. The second precipitates were washed 3 times with sodium acetate buffer at pH=5.5, to collect third supernatants and third precipitates. The radioactive activities of the third supernatants and the third precipitates were measured respectively, and the leakage rates of the boron-containing drug were calculated to be less than 1%.

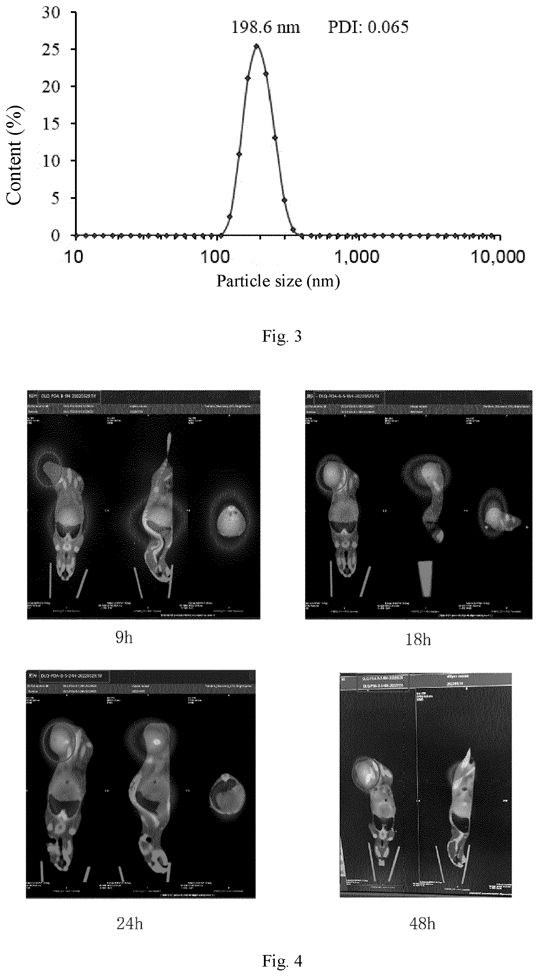

The SEM image of the ⁹⁰Y-labeled boron-containing drug is shown in Fig. 2, and the DLS image is shown in Fig. 3.

### Example 5: Preparation of Radiopharmaceuticals

This example is used to illustrate the labeling process of ¹⁷⁷Lu: 5mg of the boron-containing drug of Example 1 was weighed, added to 2.5ml of sodium acetate buffer solution at pH=5.5, and mixed uniformly with ultrasound. 500µCi of ¹⁷⁷LuCI₃ standard solution was added, and the shaking reaction was performed at 50°C in water bath for 50 minutes. Centrifugation was performed to separate a supernatant from a precipitate, the precipitate was wash 3 times with sodium acetate buffer at pH=5.5 to collect a first supernatant and a first precipitate. The radioactive activity of the first supernatant and the first precipitate were measured respectively, and the labeling rate of the boron-containing drug was calculated to be greater than 99%. The first precipitate was divided into three parts and placed in normal saline, phosphate buffer, and 5% fetal bovine serum, respectively, and incubated at 37°C for 6 days, and then centrifuged to separate second supernatants from second precipitates. The second precipitates were washed 3 times with sodium acetate buffer at pH=5.5, to collect third supernatants and third precipitates. The radioactive activities of the third supernatants and the third precipitates were measured respectively, and the leakage rates of the boron-containing drug were calculated to be less than 1%.

### Example 6: Preparation of Radiopharmaceuticals

This example is used to illustrate the labeling effects of ⁶⁴Cu, ⁶⁷Cu, ⁸⁹Zr, ⁸⁹Sr, ^{111I}n, ¹⁶⁵Dy, ¹⁶⁶Ho, ²⁰¹TI, ²¹³Bi, ²¹²Bi, ²²⁵Ac, ²²³Ra, and ²¹²Pb. The labeling process refers to Example 4. The labeling rates and leakage rates are shown in Table 1.

**Table 1: Labeling Rates and Leakage Rates of Nanoparticles Labeled with Metal Radionuclides**

| Radionuclides | ⁶⁴Cu | ⁶⁷Cu | ⁸⁹Zr | ⁸⁹Sr | ¹¹¹In | ¹⁶⁵Dy | ¹⁶⁶Ho | ²⁰¹TI | ²¹³Bi | ²¹²Bi | ²²⁵Ac | ²²³Ra | ²¹²Pb |
|---|---|---|---|---|---|---|---|---|---|---|---|---|---|
| Labeling rate/% | 95 | 95 | 94 | 98 | 99 | 99 | 98 | 95 | 70 | 70 | 81 | 96 | 83 |
| Leakage rate in normal saline for 6 days /% | 0.81 | 0.96 | 0.12 | 0.10 | 0.32 | 1.10 | 1.23 | 2.45 | 3.06 | 4.00 | 0.56 | 0.78 | 1.37 |
| Leakage rate in phosphate buffer for 6 days /% | 1.03 | 1.12 | 0.93 | 0.56 | 0.99 | 1.32 | 1.78 | 3.02 | 4.98 | 9.80 | 0.99 | 1.36 | 2.82 |

### Example 7: Preparation of Radiopharmaceuticals

This example is used to illustrate the labeling process of ¹³¹I: Chloramine T was dissolved in an organic solvent and applied to the bottom of the tube, and then dried. 25mg of the boron-containing drug of Example 1 was dispersed uniformly in 10ml of PBS buffer solution and placed in a reaction tube. The reaction tube was placed in an ice bath, and 5mCi of ¹³¹I solution was added to react for 10 minutes. Ultrafiltration was performed, and then the activity of the ultrafiltration tube and the total activity of the ultrafiltration tube and the filtrate were measured. The activity of the ultrafiltration tube/the total activity is the labeling rate, which is greater than 95%. The precipitate in the ultrafiltration tube was divided into three parts and placed in normal saline, phosphate buffer, and 5% fetal bovine serum, respectively, and incubated at 37°C for 48 hours, and then centrifuged to separate first supernatants from first precipitates. The first precipitates were washed 3 times with sodium acetate buffer at pH=5.5, to collect second supernatants and second precipitates. The radioactive activities of the second supernatants and the second precipitates were measured respectively, and the leakage rates of the boron-containing drug were calculated to be less than 1%.

### Example 8: Preparation of Radiopharmaceuticals

This example is used to illustrate the labeling process of ¹²³I: Chloramine T was dissolved in an organic solvent and applied to the bottom of the tube, and then dried. 5mg of the boron-containing drug of Example 1 was dispersed uniformly in 2ml of PBS buffer solution and placed in a reaction tube. The reaction tube was placed in an ice bath, and 1mCi of ¹²³I solution was added to react for 10 minutes. Ultrafiltration was performed, and then the activity of the ultrafiltration tube and the total activity of the ultrafiltration tube and the filtrate were measured. The labeling rate is 86%.

### Example 9: Preparation of Radiopharmaceuticals

This example is used to illustrate the labeling process of ¹⁸F: 2.5mg of the boron-containing drug was dissolved in a mixture of 0.6ml of trifluoroacetic acid and 0.9ml of chloroform to obtain a solution. A certain activity of [¹⁸F]F₂ gas and neon gas were injected into the solution, the trifluoroacetic acid and 0.9ml of chloroform were evaporated under a neon atmosphere, the residual nanoparticles were collected, the radioactive activity was measured, and the labeling rate was calculated to be 71%.

### Example 10: Preparation of Radiopharmaceuticals

This example is used to illustrate the labeling process of ^{99m}Tc: 4mg of sodium ascorbate and 1mCi of Na^{99m}TcO₄ were sequentially added to 5ml of sodium acetate buffer solution containing 5mg of nanoparticles (pH 4.0), and shook at 80°C for 15 minutes to achieve the target radionuclide labeling. The labeling rate is 85%.

### Example 11: In vivo Distribution Experiment of Boron-Containing Drug in a Mouse Subcutaneous Brain Glioma Model

The boron-containing drug of Example 1 was taken and injected into tumor-bearing mice via the tail vein with a dosage of 150mg/kg. At 12h, 24h, and 36h after administration, the heart, liver, spleen, lungs, kidneys, blood, brain, tumor, and normal tissue at the tumor margin of the mice were collected, weighed, and digested. The boron concentration in each organ was measured using ICP-MS. At 24h, the boron concentration at the tumor site reaches 74µg/g, with a T/N ratio as high as 70:1. The distribution of boron in mice at 12h, 24h, and 36h after administration is shown in Table 2.

**Table 2: Distribution of Boron in Mice at 12h, 24h, and 36h after Administration**

| Sites | B element content at different times (µG/G) | | |
|---|---|---|---|
| | 12h | 24h | 36h |
| Heart | 0.08189 | 0.17047 | 0.04152 |
| Liver | 0.13816 | 0.11147 | 0.12833 |
| Spleen | 0.29927 | 0.31259 | 0.00946 |
| Lungs | 0.29131 | 0.24431 | 0.16358 |
| Kidneys | 0.12804 | 0.05531 | 0.07213 |
| Blood | 0.51842 | 0.92589 | 0.09688 |
| Brain | 0.31613 | 0.0797 | 0.10611 |
| Tumor | 18.1967 | 74.0081 | 33.2075 |
| Normal tissue at the tumor margin | 0.54357 | 0.73641 | 0.26313 |

This example investigates the distribution in subcutaneous brain glioma model animals, where the boron-containing drug is significantly taken up at the tumor site.

### Example 12: SPECT-CT Experiment

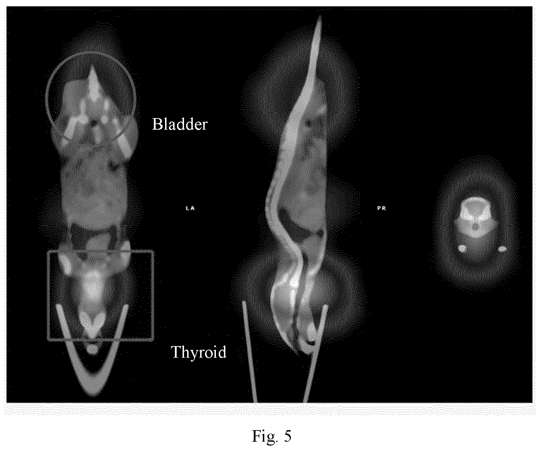

About 0.2mCi of the ¹³¹I-labeled boron-containing drug of Example 7 was injected into tumor-bearing mice via the tail vein. After the injection, static acquisitions were performed for 10 minutes at 9h, 18h, 24h, and 48h to obtain SPECT-CT image data, as shown in Fig. 4. Free ¹³¹I was injected into tumor-bearing mice via the tail vein, and SPECT-CT image data as shown in Fig. 5 were obtained from a 10-minute static acquisition. The radionuclide was primarily metabolized by the kidneys and bladder, with no significant uptake in normal tissues except for the main metabolic organs such as gallbladder, kidneys, and bladder. The tumor site showed significant uptake, indicating that the radionuclide labeling did not affect the uptake of the boron-containing drug at the tumor site.

### Example 13: Treatment of Subcutaneous Brain Glioma in Mice with ¹³¹I-Labeled Boron-Containing Drug

Normal saline and about 2mCi of the ¹³¹I-labeled boron-containing drug of Example 7 were injected into tumor-bearing mice via the tail vein, respectively. The volume of subcutaneous brain glioma in the mice was measured on days 3, 5, and 10. The specific results are shown in Table 3. The ¹³¹I-labeled boron-containing drug significantly inhibited tumor growth.

**Table 3: Volume of Subcutaneous brain Glioma in Mice**

| Time | Normal saline group | ¹³¹I-labeled boron-containing drug group |
|---|---|---|
| Day 3 | 96mm³ | 110mm³ |
| Day 5 | 487mm³ | 67mm³ |
| Day 10 | 1980mm³ | 45mm³ |

Finally, it should be noted that the above examples are provided to illustrate the technical solutions of the present invention and not to limit them. Although the present invention has been described in detail with reference to the preceding examples, those of ordinary skill in the art should understand that they can still make modifications to the technical solutions recorded in the preceding examples, or make equivalent replacements for some or all of the technical features. Such modifications or replacements do not cause the essence of the corresponding technical solutions to depart from the scope of the technical solutions of the examples of the present invention.

## Claims

1. A radionuclide-labeled boron-containing drug which is obtained by labeling a boron-containing drug by at least one radionuclide;
the boron-containing drug is obtained by polymerizing dopamine and its derivative with a boron-containing compound.

2. The radionuclide-labeled boron-containing drug according to claim 1, wherein the radionuclide comprises any one or more of ⁶⁸Ga, ⁶⁷Ga, ⁶⁴Cu, ⁶⁷Cu, ⁸⁹Zr, ⁸⁹Sr, ⁹⁰Y, ¹⁷⁷Lu, ¹¹¹In, ¹⁶⁵Dy, ¹⁶⁶Ho, ²⁰¹TI, ²¹³Bi, ²¹²Bi, ²²⁵Ac, ²²³Ra, ²¹²Pb, ²¹¹At, ¹²⁴I, ¹²³I, ¹²⁵I, ¹³¹I, ¹⁸F, ¹⁸⁶Re, ¹⁸⁸Re, and ^{99m}Tc.

3. The radionuclide-labeled boron-containing drug according to claim 1, wherein the radionuclide-labeled boron-containing drug has a particle size of 50-1000 nm.

4. The radionuclide-labeled boron-containing drug according to claim 1, wherein the boron-containing compound includes, but is not limited to, any one or more of boric acid, phenylalanine boric acid and its derivative, proline boric acid, aspartic acid boric acid, tyrosine boric acid, cysteine boric acid, methionine boric acid, serine boric acid, 5-amino-2,3-difluorophenyl boric acid, 3-amino-5-cyanophenyl boric acid, 3-amino-4-methylphenyl boric acid, 3-amino-phenylboric acid hydrochloride, and 4-carbamoyl phenylboric acid.

5. The radionuclide-labeled boron-containing drug according to claim 1, wherein the dopamine and its derivative comprise any one or more of dopamine, 4-(2-aminopropyl)phenyl-1,2-diol, norepinephrine, L-methyldopa, droxidopa, and 5-hydroxydopamine.

6. The radionuclide-labeled boron-containing drug according to claim 5, wherein the dopamine and its derivative are dopamine, L-methyldopa, or 5-hydroxydopamine.

7. The radionuclide-labeled boron-containing drug according to claim 1, wherein the weight ratio of the dopamine and its derivative to the boron-containing compound is (1-10) : 1.

8. The radionuclide-labeled boron-containing drug according to any one of claims 1-7, wherein an indication for the radionuclide-labeled boron-containing drug comprises head and neck tumors, lung cancer, peritoneal cancer, liver cancer, gastric cancer, melanoma, pancreatic cancer, glioblastoma, cervical cancer, ovarian cancer, bladder cancer, breast cancer, colon cancer, colorectal cancer, uterine cancer, salivary gland cancer, kidney cancer, prostate cancer, vulvar cancer, or thyroid cancer.

9. A method for preparing a radionuclide-labeled boron-containing drug according to any one of claims 1-8, comprising steps of: performing complexation of polyhydroxy or carboxyl groups on a boron-containing drug with a radionuclide, and performing precipitation and solidification; or
labeling a radionuclide in a phenyl ring structure of boron-containing drug by a formation of covalent bonds through chemical reactions.

10. Use of a radionuclide-labeled boron-containing drug according to any one of claims 1-8 in the preparation of a medicament for tumor treatment and/or tumor imaging.
